(19) Europäisches Patentamt
European Patent Office
Office européen des brevets

(11) Publication number: **0 387 681**
**A2**

(12) **EUROPEAN PATENT APPLICATION**

(21) Application number: **90104328.1**

(51) Int. Cl.$^5$: **C08B 37/16, A61K 31/73**

(22) Date of filing: **07.03.90**

(30) Priority: **09.03.89 IT 1970089**

(43) Date of publication of application:
**19.09.90 Bulletin 90/38**

(84) Designated Contracting States:
**AT BE CH DE DK ES FR GB GR IT LI LU NL SE**

(71) Applicant: **FARMHISPANIA S.A.**
**Primero de Mayo s/n**
**E-08080 Montmelo (Barcelona)(ES)**

(72) Inventor: **Butelman, Federico**
**Viale Bianca Maria, 19**
**I-20122 Milan(IT)**

(74) Representative: **Gervasi, Gemma et al**
**NOTARBARTOLO & GERVASI Srl Viale**
**Bianca Maria 33**
**I-20122 Milan(IT)**

(54) **Ammonium salts of polycyclodextrins for use as hypocholesteremic agents.**

(57) Ammonium salts of polycyclodextrins prepared by:

a) cross-linking the cyclodextrin;

b) esterifying the cross-linked cyclodextrin and treating the ester obtained with a dialkylamine followed by quaternization of the amino nitrogen atom.

Said salts have elevated hypocholesteremic activity and the advantage of very low toxicity.

EP 0 387 681 A2

# AMMONIUM SALTS OF POLYCYCLODEXTRINS FOR USE AS HYPOCHOLESTEREMIC AGENTS

## Field of the invention

This invention relates to compounds for the preparation of pharmaceutical compositions suitable for use as hypocholesteremic agents.

## Prior art

The hypocholesteremic action of cyclodextrins, polymerized cyclodextrins and their derivatives is well known.

For example, in patent JP 740 418, 6-methylamino-6-deoxy-cyclodextrin is described as a hypocholesteremic agent, and according to patent JP 770 330 poly(6-aminoalkyl-6-deoxy)-cyclodextrins is stated to be able to reduce the total cholesterol level of chickens by 7-20%.

However in the known art the substituent groups are generally non-ionic groups and are therefore not able to significantly interact with the bile salts other than in connection with the capacity of cyclodextrins to form inclusion complexes.

In some cases positive charges are present in the molecule due to protonization of the amino group, however under the pH conditions of the intestinal tract the proton is removed and the capacity of polycyclodextrins to interact with bile salts is very low.

## Summary of the invention

we have now discovered new compounds which are able to interact with bile salts and to exercise high hypocholesteremic activity while having the advantage of very low toxicity.

Said compounds are ammonium salts of polycyclodextrins of the following general formula:

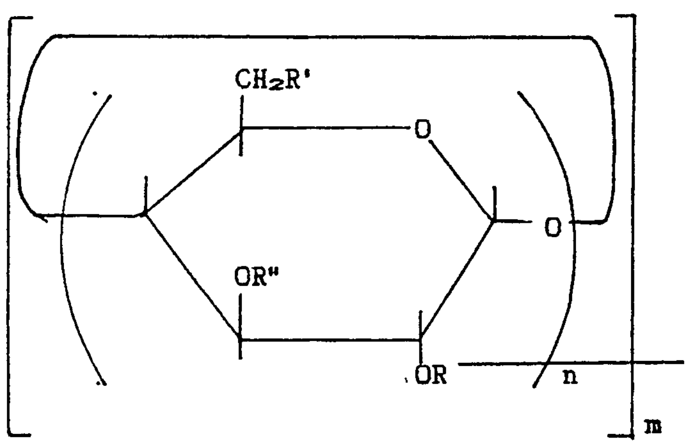

(I)

in which:

R is H or a radical of a bifunctional or polyfunctional compound which combines with one or more hydroxyl groups of the cyclodextrin; R′ is H or

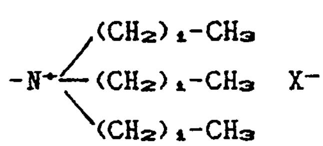

or

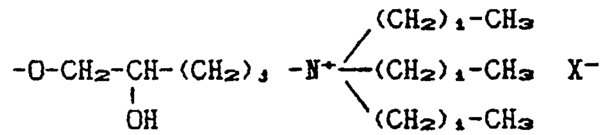

in which i is zero or a whole number from 1 to 3,
j is a whole number from 1 to 4, and X is Cl, Br, I, F, $HSO_4$ or $CH_3$-O-$SO_3$, or R′ can sometimes be the same as R; R″ is H or

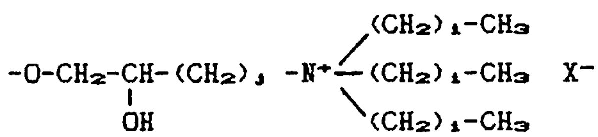

in which j and X have the aforesaid meaning;
n is a whole number from 6 to 8;
m (degree of polymerization) is a whole number from 5 to 20.

The compounds of formula (I) are prepared from cyclodextrins by a process characterised by:

a) subjecting the cyclodextrin to cross-linking;

b) esterifying the cross-linked cyclodextrin and treating the ester obtained with a dialkylamine the nitrogen atom of which is then quaternized.

Stages a) and b) can also be implemented in the following alternative manner: the quaternized product can be obtained directly by treating the product of stage a) with glycidyltrimethylammonium-chloride; the sequence of stages a) and b) can be reversed.

## Detailed description of the invention

The characteristics and advantages of the compounds of the present invention, their therapeutic application and the process for their preparation will be further illustrated in the course of the following detailed description.

The present invention relates to ammonium salts of polycyclodextrins of general formula (I) which are prepared by a process comprising:

a) cross-linking the cyclodextrin;

b) esterifying the cross-linked cyclodextrin and then aminating and quaternizing it.

This process can be implemented in various alternative ways which will be described hereinafter.

The cyclodextrin is cross-linked using as cross-linking agent compounds such as aldehydes, ketones, isocyanates, epoxides and polyols, under conditions chosen according to the characteristics of the cross-linking compound.

A preferred cross-linking compound is epichlorohydrin, with which the cross-linking of the cyclodextrin is conducted in dimethylsulphoxide and dimsyl at 40-60 °C.

Another preferred cross-linking compound is 1,4-butanediol diglycylether, with which cross-linking is conducted in isopropyl alcohol at 70-90 °C.

The molar ratio of cross-linking agent to cyclodextrin is between 0.2 and 3.0.

The cross-linked cyclodextrin is then esterified preferably with p-toluenesulphonylchloride and then aminated preferably with dimethylamine, in an organic medium, preferably methanol, at a temperature of between 75 °C and 85 °C.

The molar ratio of acid chloride to cross-linked cyclodextrin is between 1.0 and 3.0, and the molar ratio of esterified cyclodextrin to amine is between 1.0 and 10.0.

The aminated product is finally treated with an alkyl halide, preferably methyl iodide, in $CH_2Cl_2$ as medium, in the presence of triethylamine at ambient temperature.

The molar ratio of alkyl halide to aminated product is between 1.5 and 5.0.

Alternatively, the compounds (I) are prepared by treating the cross-linked cyclodextrin of stage a) with glycidyltrimethyl ammonium chloride in the presence of an alkaline catalyst.

For this purpose the cross-linking cyclodextrin of stage a) is dispersed in a mixture of isopropyl alcohol and water, an NaOH or KOH solution is added, after which the glycidyltrimethylammonium chloride is added and reacted at a temperature of between 70 °C and 90 °C.

The molar ratio of alkaline base to cyclodextrin is between 1.0 and 3.0 and the molar ratio of glycidyltrimethylammonium chloride to cyclodextrin is between 2.0 and 6.0.

According to a further alternative the compounds of the present invention can be prepared by implementing stage a) after stage b).

In all cases the degree of polymerization obtained is between 5 and 20 and the degree of substitution with amino groups is between 0.7 and 2.0.

In characterising the polycyclodextrin, the swelling and water retention can be easily determined, and elementary and infrared analyses can be carried out.

After the quaternizing reaction the molecular weight of the soluble product can be determined either by gel chromatography on Sephadex G-50 fine gels and Ultragel AcA54, or by exclusion chromatography using polysaccharides as standard.

$^{13}C$-NMR spectroscopy is used to determine the position of the bonds between the cyclodextrin molecules and the position of the amino groups in the cyclodextrin polymer.

For example, when using epichlorohydrin as cross-linking agent, the cross-linking reaction mainly involves the OH-2 and OH-3 hydroxyl groups, whereas the quaternizing reaction involves the OH-6 groups.

The available cavities in the polycyclodextrins can be determined by spectrophotometry using m-chlorobenzoic acid or a dye such as congo red or methyl orange as guest molecule model.

The compounds according to the invention are able to interact with bile salts both in the form of inclusion complexes in the cyclodextrin cavities and at the ionic interaction level as they are stable within a wide pHrange (1.5-12.0). They were used in a series of pharmacological trials on rats and the results were compared with those obtained using non-quaternized derivatives of polycyclodextrins and with cholestyramine which is the most effective currently known resin of anticholesteremic activity, as reported hereinafter.

Pharmacological trials

The pharmacological trials were conducted on 10 groups of Sprague-Dawley rats of average weight 220 g. The method of treatment and the results are shown in Table 1.

All the rats were fed with Nath diet (Nath et al, J. Nutrit. 67, 289, 1959) for 30 days with the exception of group 1 which was fed with normal diet for control purposes.

Hypercholesterolemia was induced in the rats of groups 2 to 10 by administering 1 g/day per animal of cholesterol by gastric probe, whereas group 1 was not treated.

A hypocholesteremic substance was also administered to the rats of groups 3 to 10.

After a period of 30 days all the animals were killed and the total cholesterol collected from the carotid arteries was determined by the method described by Pearson et al. (J. Chem. Endocrin. Metabolism 12, 1245, 1952).

TABLE 1

| Group | Diet and treatment | Total serous cholesterol (mg/100 ml) | Reduction in total serous cholesterol (%) |
|---|---|---|---|
| 1 | Normal diet (controls) | 95 ± 7 | 0 |
| 2 | Nath diet | 280 ± 10 | 0 |
| 3 | Nath diet + 2% cholestyramine | 145 ± 9 | 48 |
| 4. | Nath diet + 2% chitosan ET 504 U.S.P. 4.436.731 | 110 ± 5 | 60 |
| 5. | Nath diet + 2% chitosan ET 1020 EPA 86108708.8-2107 | 100 ± 6 | 65 |
| 6. | Nath diet + 2% (6-dimethylamino-6-deoxy)-β-cyclodextrin | 150 ± 7 | 47 |
| 7. | Nath diet + 2% poly(6-amino-6-deoxy) β-cyclodextrin | 120 ± 7 | 55 |
| 8. | Nath diet + 2% poly(6-amino-6-deoxy) β-cyclodextrin acetate | 95 ± 5 | 66 |
| 9. | Nath diet + 2% poly(6-ammonium-6-deoxy) β-cyclodextrin chloride | 75 ± 4 | 85 |
| 10. | Nath diet + 2% poly(6-ammonium-6-deoxy) α-cyclodextrin chloride | 77 ± 5 | 83 |
| 11. | Nath diet + 2% cross-linked dextran having quaternary ammino group (example 1 EPA No. 66153) | | 42 |

Besides presenting high hypocholesteremic activity, the compounds according to the present invention are substantially free of toxicity on oral administration, and the charge density in the macromolecules and their molecular weight prevent permeation of the intestinal wall. The cross-linking of the monomer units together with their derivative formation with ammonium groups prevents enzymatic attack by α-amylase.

The compounds of the present invention can be used in the preparation of pharmaceutical compounds suitable for the treatment of hypercholesterolemia, in association with diluent and excipient substances normally used in the pharmaceutical field.

EXAMPLE 1

1.5 g of NaH and 15 ml of epichlorohydrin are added under stirring at 50 °C to a solution of 50 g of β-cyclodextrin in 500 ml of dimethylsulphoxide.

The mixture is stirred for 10 hours, and after cooling to ambient temperature is diluted with acetone, the solid product being filtered off and washed with methanol.

The product is dispersed in 300 ml of an isopropyl alcohol/water mixture (80:20 v/v) and 10 g of NaOH dissolved in 50 ml of water are added.

After 30 minutes 70 g of glycidyltrimethylammonium chloride are added and the mixture is kept stirring at 80 °C for 16 hours.

After cooling to ambient temperature the mixture is diluted with petroleum ether and the insoluble product is filtered off and washed with acetone. 46.34 g of dry product are obtained.

The degree of substitution of the hydroxyl groups by ammonium groups, determined by $^1$H-NMR, is 0.8, and the degree of β-cyclodextrin polymerization determined by exclusion chromatography is 20.

EXAMPLE 2

50 g of β-cyclodextrin are dissolved in 500 ml of water, and 150 g of glycidyltrimethylammonium chloride and 2.0 g of NaOH are added.

The mixture is stirred for 4 hours at ambient temperature after which 3 g of NaOH are added to obtain the mixture (A).

Separately, 50 ml of toluene, 10 g of an emulsifying agent and Calofort$^R$S are mixed together to obtain the mixture (B), this being heated to 70 °C and added to the mixture (A) which had been previously heated to 70 °C.

The resultant mixture is stirred for 30 minutes, 15 ml of epichlorohydrin are added and stirring continued for 8 hours.

After cooling to ambient temperature the mixture is poured into 500 ml of isopropyl alcohol, stirred for 15 minutes, and after sedimentation the solid product is filtered off and washed firstly with an ethanol/HCl mixture and then with methanol.

45 g of product are obtained having a degree of substitution of 0.95, determined by $^1$H-NMR, and a degree of β-cyclodextrin polymerization of 10, determined by exclusion chromatography.

EXAMPLE 3

6 g of p-toluenesulphonyl chloride are added to a solution of 10 g of β-cyclodextrin in 75 ml of pyridine.

The mixture is stirred at ambient temperature for 16 hours and is then poured into a water/ice mixture.

The precipitate obtained is filtered off, washed with water and dried under vacuum, to obtain 15.6 g of 6-O-tosyl-β-cyclodextrin.

This product is dissolved in 150 ml of a 20% w/v solution of dimethylamine.

The mixture is heated to 80 °C for 16 hours in a closed reactor, and after cooling to ambient temperature the solvent is evaporated to dryness.

The product obtained (7.2 g) is dispersed in $CH_2Cl_2$ and treated with 3.5 ml of methyl iodide and 2 ml of triethylamine while stirring at ambient temperature.

Stirring is continued for 4 hours and the reaction mixture is then filtered, the solvent being evaporated to obtain a crude product consisting of 5.8 g of (6-O-ammonium-6-deoxy)-β-cyclodextrin iodide.

This product is dissolved in 50 ml of water and ion-exchanged with $Cl^-$ using Dowex 1 x 8 resin in $Cl^-$

form.

1.5 g of NaOH and 10 g of 1,4-butanedioldiglycidylether in 30 ml of isopropyl alcohol are added. The temperature is raised to 80°C and the reaction mixture stirred for 12 hours and then processed as in Example 2.

9.2 g of dry product are obtained with a degree of ammonium group substitution of 0.7 and a degree of β-cyclodextrin polymerization of 12.

## Claims

1. Ammonium salts of polycyclodextrins of the following general formula:

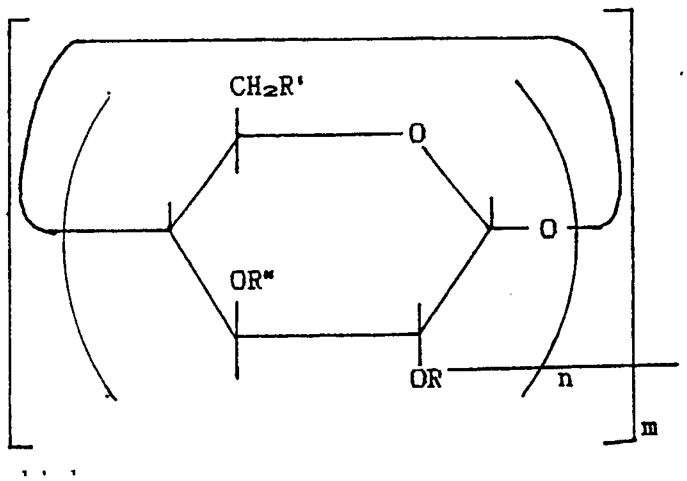

(I)

in which:

R is H or a radical of a bifunctional or polyfunctional cross-linking agent; R′ is OH or

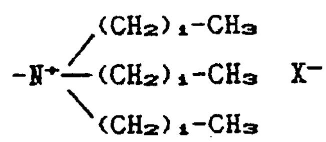

or

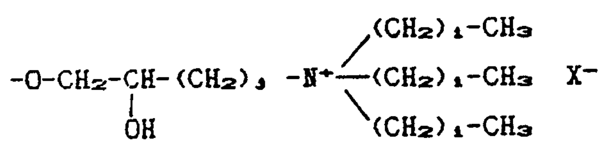

in which i is zero or a whole number from 1 to 3,

j is a whole number from 1 to 4, and X is Cl, Br, I, F, $HSO_4$ or $CH_3$-O-$SO_3$, or R′ can sometimes be identical to R;

R″ is H or

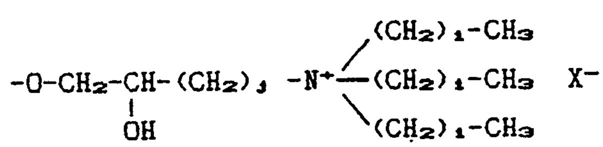

in which j and X have the aforesaid meaning;
n is a whole number from 6 to 8;
m is a whole number from 5 to 20.

2. Salts as claimed in claim 1, characterised in that the degree of substitution by ammonium groups is between 0.7 and 2.0.

3. A method for preparing ammonium salts of polycyclodextrins of the following general formula:

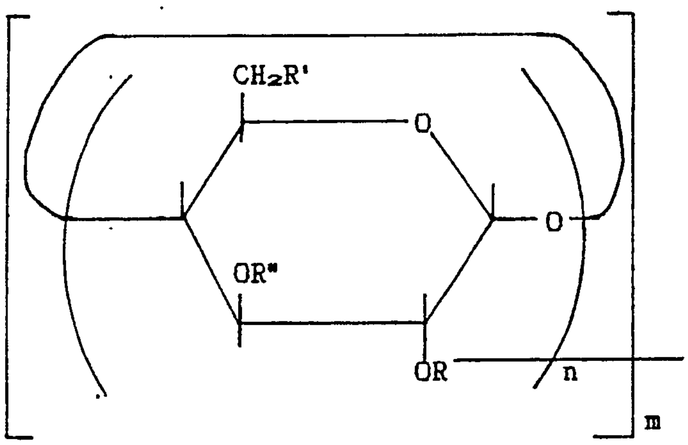

(I)

in which:
R is H or a radical of a bifunctional or polyfunctional cross-linking agent;
R′ is OH or

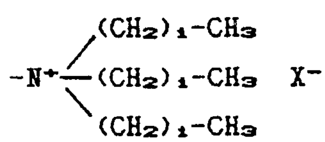

or

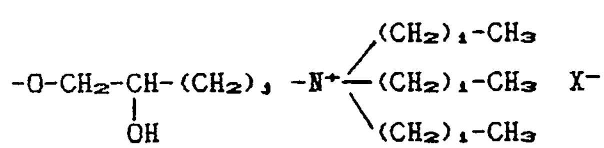

in which i is zero or a whole number from 1 to 3,
j is a whole number from 1 to 4, and X is Cl, Br, I, F, $HSO_4$ or $CH_3$-O-$SO_3$, or R′ can sometimes be identical to R;
R″ is H or

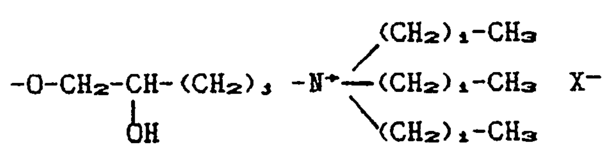

in which j and X have the aforesaid meaning;
n is a whole number from 6 to 8;
m is a whole number from 5 to 20;

characterised by:

a) subjecting the cyclodextrin to cross-linking;

b) esterifying the cross-linked cyclodextrin and treating the ester obtained with a dialkylamine the nitrogen atom of which is then quaternized.

4. A method as claimed in claim 3, characterised in that the quaternized product is obtained directly by treating the product of stage a) with glycidyl trimethylammonium chloride.

5. A method as claimed in claim 3, characterised in that the sequence of stages a) and b) is reversed.

6. A method as claimed in claim 3, characterised in that compounds such as aldehydes, ketones, isocyanates, epoxides and polyols are used as cross-linking agents.

7. A method as claimed in claim 6, characterised in that epichlorohydrin and 1,4-butanedioldiglycidylether are used as cross-linking agents.

8. A method as claimed in claim 3, 'characterised in that said cross-linking is conducted in an organic solvent at a temperature of between 40° C and 90° C.

9. A method as claimed in claim 3, characterised in that the molar ratio of cross-linking agent to cyclodextrin is between 0.2 and 3.0.

10. A method as claimed in claim 3, characterised in that said esterification is conducted with p.toluenesulphonylchloride, with a molar ratio of this latter to cross-linked cyclodextrin of between 1.0 and 3.0.

11. A method as claimed in claim 3, characterised in that said treatment with dialkylamine is conducted in an organic solvent at a temperature of between 75° C and 85° C.

12. A method as claimed in claim 3, characterised in that said treatment of the cyclodextrin ester with dialkylamine is conducted with a molar ratio of said ester to said amine of between 1.0 and 10.

13. A method as claimed in claim 3, characterised in that said quaternization is effected by treating the aminated product with an alkyl halide in $CH_2Cl_2$ in the presence of triethylamine at ambient temperature.

14. A method as claimed in claim 13, characterised in that the molar ratio of alkyl halide to aminated product is between 1.5 and 5.

15. A method as claimed in claim 4, characterised in that said treatment of the product of stage a) with glycidyl trimethylammonium chloride is conducted in a mixture of isopropyl alcohol and water in the presence of an alkaline base at a temperature of between 70° C and 90° C.

16. A method as claimed in claim 15, characterised in that the ratio of glycidyl trimethylammonium chloride to cyclodextrin is between 2.0 and 6.0.

17. The use of ammonium salts of polycyclodextrins of general formula (I) in the preparation of pharmaceutical compounds suitable for the treatment of hypercholesterolemia, in association with diluent and excipient substances normally used in the pharmaceutical field.

Claims for the following Contracting States: GR, ES

1. A method for preparing ammonium salts of polycyclodextrins of the following general formula:

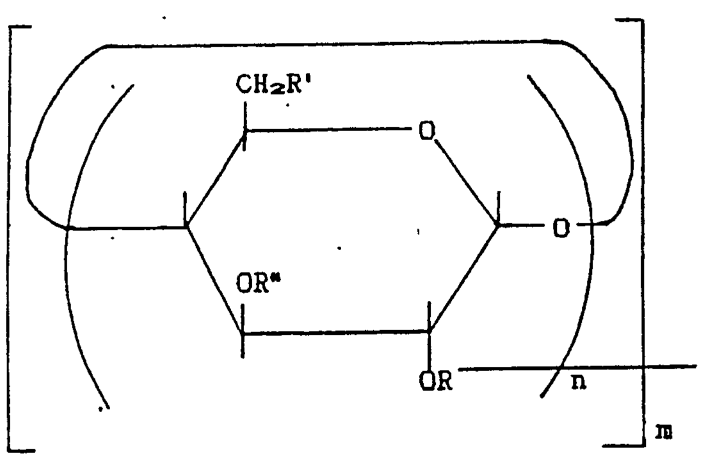

(I)

in which:

R is H or a radical of a bifunctional or polyfunctional cross-linking agent:

R′ is OH or

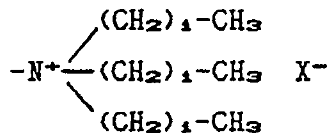

or

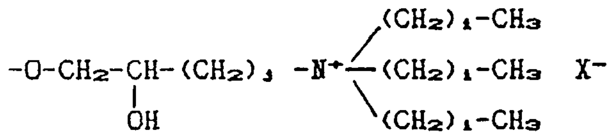

in which i is zero or a whole number from 1 to 3,
j is a whole number from 1 to 4, and X is Cl, Br, I, F, $HSO_4$ or $CH_3$-O-$SO_3$, or R′ can sometimes be identical to R;
R″ is H or

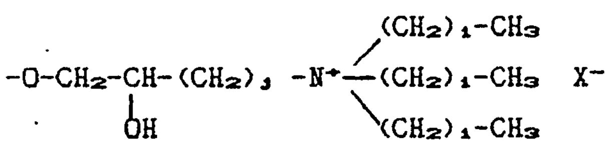

in which j and X have the aforesaid meaning;
n is a whole number from 6 to 8;
m is a whole number from 5 to 20;
characterised by:

a) subjecting the cyclodextrin to cross-linking;

b) esterifying the cross-linked cyclodextrin and treating the ester obtained with a dialkylamine the nitrogen atom of which is then quaternized.

2. A method as claimed in claim 1, characterised in that the quaternized product is obtained directly by treating the product of stage a) with glycidyl trimethylammonium chloride.

3. A method as claimed in claim 1, characterised in that the sequence of stages a) and b) is reversed.

4. A method as claimed in claim 1, characterised in that compounds such as aldehydes, ketones, isocyanates, epoxides and polyols are used as cross-linking agents.

5. A method as claimed in claim 4, characterised in that epichlorohydrin and 1,4-butanedioldiglycidylether are used as cross-linking agents.

6. A method as claimed in claim 1, characterised in that said cross-linking is conducted in an organic solvent at a temperature of between 40° C and 90° C.

7. A method as claimed in claim1, characterised in that the molar ratio of cross-linking agent to cyclodextrin is between 0.2 and 3.0.

8. A method as claimed in claim 1, characterised in that said esterification is conducted with p. toluenesulphonylchloride, with a molar ratio of this latter to cross-linked cyclodextrin of between 1.0 and 3.0.

9. A method as claimed in claim 1, characterised in that said treatment with dialkylamine is conducted in an organic solvent at a temperature of between 75° C and 85° C.

10. A method as claimed in claim 1, characterised in that said treatment of the cyclodextrin ester with dialkylamine is conducted with a molar ratio of said ester to said amine of between 1.0 and 10.

11. A method as claimed in claim 1, characterised in that said quaternization is effected by treating the aminated product with an alkyl halide in $CH_2Cl_2$ in the presence of triethylamine at ambient temperature.

12. A method as claimed in claim 11, characterised in that the molar ratio of alkyl halide to aminated product is between 1.5 and 5.

13. A method as claimed in claim 2, characterised in that said treatment of the product of stage a) with glycidyl trimethylammonium chloride is conducted in a mixture of isopropyl alcohol and water in the presence of an alkaline base at a temperature of between 70° C and 90° C.

14. A method as claimed in claim 13, characterised in that the ratio of glycidyl trimethylammonium chloride to cyclodextrin is between 2.0 and 6.0.

15. The use of ammonium salts of polycyclodextrins of general formula (I) in the preparation of pharmaceutical compounds suitable for the treatment of hypercholesterolemia, in association with diluent and excipient substances normally used in the pharmaceutical field.